# EUROPÄISCHE PATENTSCHRIFT

(11) **EP 0 438 767 B1**
(45) Veröffentlichungstag und Bekanntmachung des Hinweises auf die Patenterteilung: **05.07.1995**
(21) Anmeldenummer: 90125316.1
(22) Anmeldetag: 22.12.1990
(51) Int. Cl.: C12P 25/00

(54) **Verfahren zur Abtrennung von Riboflavin aus Fermentationssuspensionen**
Process for the separation of riboflavin from a fermentation suspension
Procédé de séparation de riboflavine d'une suspension de fermentation

(30) Priorität: 25.01.1990 DE 4002066
(43) Veröffentlichungstag der Anmeldung: 31.07.1991
(73) Patentinhaber: BASF Aktiengesellschaft, 67063 Ludwigshafen (DE)
(72) Erfinder: Faust, Tillmann, Dr., W-6714 Weisenheim (DE); Meyer, Joachim, Dr., W-6717 Hessheim (DE); Wellinghoff, Georg, Dr., W-6800 Mannheim (DE); Goesele, Walter, Dr., W-6900 Heidelberg (DE); Martin, Christoph, Dr., W-6800 Mannheim 1 (DE); Grimmer, Johannes, W-6700 Ludwigshafen (DE)

(56) Entgegenhaltungen:
- EP-A- 0 341 433
- DE-A- 1 925 001
- DE-A- 2 920 592

## Beschreibung

Die vorliegende Erfindung betrifft ein verbessertes Verfahren zur Abtrennung von Riboflavin aus Fermentationssuspensionen durch Zentrifugieren.

Aus der DE-C 2 920 592 ist ein Verfahren zur Abtrennung von Riboflavin aus Fermentersuspensionen bekannt, bei welchem die Fermentationssuspensionen mit 25 bis 100 Vol-% wasser verdünnt und anschließend für 15 bis 45 Minuten auf 50 bis 65°C erwärmt werden. Nach dem Abkühlen der Suspensionen werden diese zur Anreicherung von Riboflavin zweimal zentrifugiert. Infolge der Verdünnung erhält man ein größeres aufzuarbeitendes Volumen an Fermentersuspensionen, wodurch die Kosten der Aufarbeitung und die Riboflavinverluste durch das im zugesetzten Wasser gelöste Riboflavin erhöht werden.

Aufgabe der Erfindung war es, eine Abtrennung von Riboflavin aus Fermentersuspensionen unter möglichst geringem Verlust an Riboflavin durchzuführen und dabei einen Feststoff mit einem möglichst hohen Riboflavinanteil zu erhalten.

Demgemäß wurde ein Verfahren zur Abtrennung von Riboflavin aus Fermentationssuspensionen durch Zentrifugieren gefunden, welches dadurch gekennzeichnet ist, daß man die Fermentationssuspensionen
a) für 1 bis 3 Stunden auf 50 bis 90°C erwärmt, danach
b) über einen Zeitraum von 1 bis 10 Stunden auf 0 bis 30°C abkühlt, anschließend
c) durch Zentrifugieren in einer Dekantierzentrifuge, die nach dem Prinzip eines Klassierers betrieben wird, so in eine Schlammfraktion und eine Flüssigfraktion zerlegt, daß die Schlammfraktion überwiegend Riboflavinkristalle als Feststoff enthält und die Flüssigfraktion praktisch kein kristallines Riboflavin mehr enthält ,
d) gewünschtenfalls die Schlammfraktion mit 0,5 bis 2 Volumenteilen Wasser pro Volumenteil Schlammfraktion resuspendiert und den Vorgang (c) ein- oder mehrmals wiederholt.

Die Riboflavin Fermentationssuspensionen Können nach an sich bekannten verfahren (siehe z.B. EP-A 231 605, EP-A 211 289; T. Szszesniak et al., Acta Microbiologica Polonica Ser. B, (1971), Band 3 (20), Nr. 1, Seiten 29-34; dto. (1971), Band 3 (20) Nr. 2, Seiten 91-95), beispielsweise unter Verwendung von Mutanten von Hefezellen des Genus Saccharomyces, Mutanten der Stämme Candida flareri CA 18Y8-6#2 und 6A 18Y8-6#2#11 und Mutanten des Stammes Ashbya gossypii erhalten werden.

Diese Fermentationssuspensionen enthalten einen Anteil an Riboflavin von bis zu 20 Gew.-%, bezogen auf den Gesamtfeststoff der Suspensionen. Die verbleibenden Feststoffanteile bestehen im wesentlichen aus komplexen Zellbestandteilen.

Wesentlich für das erfindungsgemäße Verfahren ist die Erwärmung der Fermentersuspension, die vorzugsweise über einen Zeitraum von 1 bis 3 Stunden, insbesondere über 1 bis 2 Stunden erfolgt. Hierdurch wird eine Kristallumwandlung des Riboflavins erreicht, bei der sich überwiegend größere Kristalle auf Kosten der Kleineren bilden.

Die Umwandlungstemperatur liegt vorzugsweise zwischen 55 und 80°C, insbesondere zwischen 60 und 75°C.

Die Abkühlung der Fermentationssuspension auf 0 bis 30°C erfolgt vorzugsweise über einen Zeitraum von 1 bis 8 Stunden, insbesondere 1 bis 5 Stunden. Hierdurch wird eine weitere Optimierung der Form der RiboflavinKristalle erreicht.

Durch die so erzielte Beschaffenheit der Riboflavinkristalle wird bei Verwendung geeigneter Trennvorrichtungen eine optimale Abtrennbarkeit der Kristalle von den spezifisch leichteren Komplexen Zell- und Medienbestandteilen der Fermentationssuspensionen ermöglicht, d.h. eine Auftrennung in eine als Feststoff überwiegend Riboflavinkristalle enthaltende Schlammfraktion und eine Flüssigfraktion, die praktisch kein kristallines Riboflavin mehr, aber einen großen Teil der Komplexen Zellbestandteile enthält.

Als geeignete Trennvorrichtungen zur Abtrennung des Riboflavins aus den Fermentersuspensionen kann man Zentrifugen vom Dekantertyp einsetzen, welche die Trennung in zwei Fraktionen gestatten, wenn man sie nach dem Prinzip eines Klassierers betreibt. Als Klassierer bezeichnet man Trennvorrichtungen, die eine Trübe nur in ein mehr oder weniger entwässertes Sinkgut und einen die feinen Schlämme enthaltenden Überlauf zerlegen (vgl. Winnacker, Richter, Chemische Technologie, 1984, Band 1, Seite 73 ff).

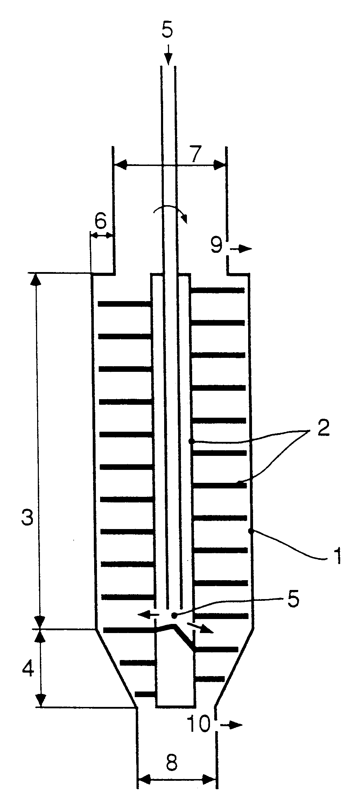

Besonders vorteilhaft gestaltet sich das Verfahren, wenn man für das Zentrifugieren der thermisch vorbehandelten Fermentersuspensionen eine Vollmantelschneckenzentrifuge vom Dekantertyp verwendet, wie sie in der Figur schematisch dargestellt ist. Hierin haben die Ziffern (1) bis (10) folgende Bedeutung:
(1) Trommel
(2) Förderschnecke
(3) Zylindrischer Sedimentationsteil
(4) Konischer Entwässerungsteil
(5) Suspensionszulauf
(6) einstellbare Überlauf-Wehrhöhe
(7) Überlaufdurchmesser
(8) Schlammaustragsdurchmesser
(9) Flüssigkeitsablauf
(10) Schlammaustrag.

Die Geometrie und die Fahrweise werden optimal auf die erfindungsgemäß umkristallisierte Riboflavin-Kristallsuspension abgestimmt. Wichtige Parameter sind vor allem die Trommelform, die Trommeldrehzahl, die Schnecken-Differenzdrehzahl, die Überlauf-Wehrhöhe (6) sowie der Suspensionsdurchsatz, d.h. die Klärflächenbelastung.

Um Schwankungen in der Riboflavinsuspension hinsichtlich Feststoffgehalt und dem Verhältnis von Riboflavin zu Zellmasse und anderen Medienbestandteilen in der Suspension zu Kompensieren, sollte die Zentrifuge eine möglichst große aktive Klassierfläche besitzen. Dies erreicht man einerseits durch Verwendung einer Trommel (1) mit erhöhtem Schlankheitsgrad (Schlankheitsgrad = Länge der Zentrifuge/Durchmesser der Zentrifuge), d.h. einem Schlankheitsgrad von 3 bis 6, vorzugsweise von 4 oder größer als 4, und andererseits durch Verschieben des Verhältnisses des zylindrischen Sedimentationsteils (3) zum konischen Entwässerungsteil (4) durch Ausbildung eines steilen Konusses in Richtung Sedimentationsteil. Mit Vorteil arbeitet man mit einem Konuswinkel von 10 bis 25°, insbesondere von 10 bis 17°.

Der Suspensionszulauf (5) erfolgt vorzugsweise etwa am Übergang vom zylindrischen zum Konischen Teil der Zentrifuge.

Auch die Überlauf-Wehrhöhe (6) des Dekanters muß an die Riboflavin-Kristallsuspension angepaßt werden. Dabei wird vorzugsweise ein Überlaufdurchmesser (7) eingestellt, der sich in einem Bereich von etwa ±10 mm vom Schlammaustragsdurchmesser (8) unterscheidet. Wird eine zu große Wehrhöhe (6) gewählt (in diesem Fall ist Überlaufdurchmesser < Schlammaustragsdurchmesser; "negatives Wehr"), so Kann es zu einem Kurzschlußartigen Austritt von Zulaufsuspension am Schlammaustrag kommen, der eine Verminderung der Produktreinheit bewirkt. Wird eine zu geringe Wehrhöhe gewählt (in diesem Fall ist Überlaufdurchmesser > Schlammaustragsdurchmesser), so tritt durch Feststoff-Rückstau im Entwässerungsteil ein erhöhter Riboflavinanteil als Verlust am Überlauf aus.

Um eine optimale Klassierwirkung zwischen den Riboflavinkristallen einerseits und dem Zellmaterial sowie den Medienbestandteilen andererseits zu erhalten, das heißt, um eine optimale Verweilzeit im Dekanter zu erreichen, müssen bei gegebener Dekantergröße die Trommeldrehzahl, die Schnecken-Differenzdrehzahl und der Suspensionsdurchsatz aufeinander abgestimmt werden. Wird z.B. bei vorgegebenem Suspensionsdurchsatz eine zu geringe Trommeldrehzahl gewählt, so tritt infolge der zu geringen Sedimentationsbeschleunigung ein erhöhter Teil an Riboflavinkristallen als Verlust am Überlauf aus. Wird die Drehzahl dagegen zu hoch gewählt, so kommt es infolge vermehrten Sedimentierens von Zellmaterial bzw. Medienbestandteilen zu einer geringeren Erhöhung der Produktreinheit.

Gegenstand der Erfindung ist daher auch ein Verfahren zur Abtrennung von Riboflavin aus Fermentationssuspensionen gemäß dem oben definierten Verfahren, das dadurch gekennzeichnet ist, daß man im Reaktionsschritt c) die Fermentationssuspension durch Zentrifugieren auf die Weise in eine Schlammfraktion und eine Flüssigfraktion zerlegt, daß der Feststoffanteil der Schlammfraktion mindestens zu 60 % aus Riboflavinkristallen besteht und die Flüssigfraktion noch einen großen Teil der komplexen Zellbestandteile enthält. Dies kann man dadurch erreichen, daß man im Reaktionsschritt c) das Zentrifugieren in einer Dekantierzentrifuge durchführt und sie nach dem Prinzip eines Klassierers betreibt. Besonders vorteilhaft ist es, wenn man im Reaktionsschritt c) das Zentrifugieren in einer Vollmantelschneckenzentrifuge vom Dekantertyp mit einem Schlankheitsgrad von gleich oder größer 4 und einem Konuswinkel von 10 bis 25° durchführt und sie nach dem Prinzip eines Klassierers betreibt, wobei der Überlaufdurchmesser gleich dem Schlammaustragsdurchmesser ± 10 mm beträgt.

Hierbei ist es besonders vorteilhaft, wenn die Schneckendrehzahl ± 0,1 % bis ± 1 % der Trommeldrehzahl und die Klärflächenbelastung (Klärflächenbelastung = Verhältnis von Suspensionsdurchsatz zu äquivalenter Klärfläche) beim ersten Dekantiervorgang 0,8 bis 1,8 l/(m² · h), vorzugsweise 1 bis 1,5 l/(m² · h) und ggf. bei einer Wiederholung des Dekantiervorgangs 0,2 bis 0,8 l/(m² · h), vorzugsweise 0,4 bis 0,6 l/(m² · h) beträgt.

In der Regel wählt man die Bedingungen des Zentrifugierens so, daß die erhaltene Schlammfraktion noch 65 bis 90 Gew.-% vorzugsweise 70 bis 85 Gew.-% Wasser enthält.

Der in den Schlammfraktionen enthaltene Feststoff besteht zu über 60 Gew.-% aus Riboflavin. Zur weiteren Steigerung des Riboflavinanteils am Gesamtfeststoffanteil kann die Schlammfraktion resuspendiert und erneut Zentrifugiert werden.

Zur Resuspendierung der Schlammfraktionen verwendet man vorzugsweise 0,5 bis 2 Volumenteile, insbesondere 0,7 bis 1,5 Volumenteile Wasser pro Volumenteil Schlamm.

Die Schlammfraktionen, die über 60 Gew.-% Riboflavin enthalten, können nach der Entwässerung direkt als Tierfutterzusatz oder nach weiterer Aufreinigung für pharmazeutische Zwecke eingesetzt werden.

Die Trocknung der Schlammfraktion kann z.B. durch Sprühwirbelschichtgranulation erfolgen.

Mit Hilfe des erfindungsgemäßen Verfahrens kann auf einfache Weise und bei geringen Riboflavinverlusten aus Fermentationssuspensionen bei einmaligem Dekantieren bis etwa 60 % reines Riboflavin und bei Wiederholung des Dekantiervorganges etwa 75 bis 88 % reines Riboflavin erhalten werden.

### Beispiel 1

Eine Fermentationssuspension, die zu etwa 85 Gew.-% aus Wasser und zu 15 Gew.-% aus Feststoff bestand, wobei der Riboflavinanteil am Feststoff etwa 17 Gew.-% betrug, wurde für zwei Stunden (h) auf 60°C erwärmt. Anschließend wurde die Fermentationssuspension über einen Zeitraum von 5 Stunden auf 20°C abgekühlt. Die so behandelte Suspension wurde mit Hilfe einer Vollmantelschneckenzentrifuge mit einem Schlankheitsgrad von 4, einem Konuswinkel von 17°, einem um 3 mm kleineren Überlaufdurchmesser als dem Schlammabwurfdurchmesser, einem Suspensionszulauf etwa am Übergang vom Zylinder der Zentrifuge zum Konus und einer Klärflächenbelastung von 1,3 l/(m² · h) auf die Weise zentrifugiert, daß die Schlammfraktion zu 20 Gew.-% aus Feststoff und 80 Gew.-% aus Wasser bestand.

Der Riboflavinanteil am Feststoff der Schlammfraktion betrug 63 Gew.-% bei Verlusten an Riboflavin von 1,8 Gew.-%.

### Beispiel 2

Eine Fermentationssuspension der in Beispiel 1 beschriebenen Zusammensetzung wurde für eine Stunde auf 75°C erwärmt. Anschließend wurde analog zu Beispiel 1 zentrifugiert.

Es wurde eine Schlammfraktion erhalten, die zu 66 Gew.-% aus Riboflavin bestand, wobei die Verluste an Riboflavin 1,9 Gew.-% betrugen.

### Beispiel 3

Die gemäß Beispiel 1 erhaltene Schlammfraktion wurde mit 0,8 Volumenteilen Wasser pro Volumenteil Schlammfraktion verdünnt und zur weiteren Aufkonzentrierung mit Hilfe einer Vollmantelschneckenzentrifuge mit einem Schlankheitsgrad von etwa 4, einem Konuswinkel von 17°, gleichem Überlauf-und Schlammabwurfdurchmesser, einem Suspensionszulauf etwa am Übergang vom zylindrischen Sedimentationsteil zum konischen Entwässerungsteil und mit einer Klärflächenbelastung von 0,5 l/(m² · h) zentrifugiert. Die Differenzdrehzahl wurde dabei so an den Zulauf angepaßt, daß gerade kein Feststoffrückstau entstand. Der Riboflavinanteil am Feststoff der erhaltenen Schlammfraktion betrug 88 Gew.-% bei Verlusten an Riboflavin von 1,0 % der in Beispiel 1 eingesetzten Riboflavin-Kristallsuspension.

### Beispiel 4

### (Vergleichsbeispiel: thermische Behandlung und Zentrifugation nicht erfindungsgemäß)

Eine Fermentationssuspension der in Beispiel 1 beschriebenen Zusammensetzung wurde analog Beispiel 1 aus DE 29 20 592 innerhalb von 30 Minuten (min) auf 60°C erwärmt, 10 min bei 60°C gehalten und innerhalb von 1 h auf 20°C abgekühlt.

Anschließend erfolgte die Aufkonzentrierung in einer Vollmantelzentrifuge mit einem Schlankheitsgrad von ca. 3, einem Konuswinkel von 10°, einer Trockenstrecke von 115 mm (bedingt durch eine Differenz von 20 mm zwischen Überlauf- und Schlammabwurfdurchmesser), einem Suspensionszulauf etwa am Übergang Zylinder/Konus und einer Klärflächenbelastung von 0,8 l/(m² · h). Der Riboflavinanteil am Feststoff der erhaltenen Schlammfraktion betrug 46 Gew.-% bei Verlusten an Riboflavin von 6,5 % der Ausgangssuspension.

### Beispiel 5

### (Vergleichsbeispiel: nur thermische Behandlung nicht erfindungsgemäß)

Eine Fermentationssuspension etwa der in Beispiel 1 beschriebenen Zusammensetzung wurde analog zu Vergleichsbeispiel 4 thermisch behandelt.

Anschließend erfolgte die Aufkonzentrierung in einer Vollmantelzentrifuge mit einem Schlankheitsgrad von ca. 3, einem Konuswinkel von 10°, Überlaufdurchmesser = Schlammabwurfdurchmesser, Suspensionszulauf etwa am Übergang Zylinder/Konus bei einer Klärflächenbelastung von 1,1 l/(m² · h). Die Differenzdrehzahl wurde so an den Zulauf angepaßt, daß kein Feststoffrückstand entstand.

Der Riboflavingehalt am Feststoff der erhaltenen Schlammfraktion betrug 58Gew.-% bei Verlusten an Riboflavin von 4,4%.

## Patentansprüche

1. Verfahren zur Abtrennung von Riboflavin aus Fermentationssuspensionen durch Zentrifugieren, **dadurch gekennzeichnet**, daß man die Fermentationssuspensionen
a) für 1 bis 3 Stunden auf 50 bis 90°C erwärmt, danach
b) über einen Zeitraum von 1 bis 10 Stunden auf 0 bis 30°C abkühlt, anschließend
c) durch Zentrifugieren in einer Dekantierzentrifuge, die nach dem Prinzip eines Klassierers betrieben wird, so in eine Schlammfraktion und eine Flüssigfraktion zerlegt, daß die Schlammfraktion überwiegend Riboflavinkristalle als Feststoff enthält und die Flüssigfraktion praktisch kein kristallines Riboflavin mehr enthält und
d) gewünschtenfalls die Schlammfraktion mit 0,5 bis 2 Volumenteilen Wasser pro Volumenteil Schlammfraktion resuspendiert und den Vorgang (c) ein- oder mehrmals wiederholt.

2. Verfahren zur Abtrennung von Riboflavin aus Fermentationssuspensionen gemäß Anspruch 1, **dadurch gekennzeichnet**, daß man im Reaktionsschritt c) die Fermentationssuspension durch Zentrifugieren auf die Weise in eine Schlammfraktion und eine Flüssigfraktion zerlegt, daß der Feststoffanteil der Schlammfraktion mindestens zu 60 % aus Riboflavinkristallen besteht und die Flüssigfraktion noch einen großen Teil der Komplexen Zellbestandteile enthält.

3. Verfahren zur Abtrennung von Riboflavin aus Fermentationssuspersionen gemäß Anspruch 1, **dadurch gekennzeichnet**, daß man im Reaktionsschritt c) das Zentrifugieren in einer Vollmantelschneckenzentrifuge vom Dekantertyp mit einem Schlankheitsgrad von gleich oder größer 4 und einem Konuswinkel von 10 bis 25° durchführt und sie nach dem Prinzip eines Klassierers betreibt, wobei der Überlaufdurchmesser gleich dem Schlammaustragsdurchmesser ± 10 mm beträgt.

4. Verfahren zur Abtrennung von Riboflavin aus Fermentationssuspensionen gemäß Anspruch 1, **dadurch gekennzeichnet**, daß im Reaktionsschritt c) das Zentrifugieren in einer Vollmantelschneckenzentrifuge vom Dekantertyp mit einem Schlankheitsgrad von gleich oder größer 4 und einem Konuswinkel von 10 bis 25° durchführt und sie nach dem Prinzip eines Klassierers betreibt, wobei die Schneckendifferenzdrehzahl ± 0,1 bis ± 1 % der Trommeldrehzahl und die Klärflächenbelastung beim ersten Dekantiervorgang 0,8 bis 1,8 l/(m² · h) und ggf. bei einer Wiederholung des Dekantiervorgangs 0,2 bis 0,8 l/(m² · h) beträgt.

5. Verfahren zur Abtrennung von Riboflavin aus Fermentationssuspensionen gemäß Anspruch 5, **dadurch gekennzeichnet**, daß die Klärflächenbelastung beim ersten Dekantiervorgang 1 bis 1,5 l/(m² · h) und ggf. bei einer Wiederholung des Dekantiervorganges 0,4 bis 0,6 l/(m² · h) beträgt.

## Claims

1. A process for removing riboflavin from fermentation suspensions by centrifugation, which comprises the fermentation suspensions
a) being heated at from 50 to 90°C for from 1 to 3 hours and then
b) being cooled to from 0 to 30°C over a period of from 1 to 10 hours, and subsequently
c) being centrifuged in a decanting centrifuge operated on the classification principle to give a sediment fraction and a liquid fraction in such a way that the sediment fraction contains mainly riboflavin crystals as solid, and the liquid fraction contains virtually no crystalline riboflavin,
d) if desired resuspending the sediment fraction with from 0.5 to 2 parts by volume of water per part by volume of sediment fraction, and repeating procedure (c) one or more times.

2. A process for removing riboflavin from fermentation suspensions as claimed in claim 1, which comprises the fermentation suspension being centrifuged in step c) to give a sediment fraction and a liquid fraction so that at least 60 % of the solids in the sediment fraction is composed of riboflavin crystals, and the liquid fraction still contains a large part of the complex cellular constituents.

3. A process for removing riboflavin from fermentation suspensions as claimed in claim 1, wherein the centrifugation in step c) is carried out in a decanter-type solid-bowl centrifuge with a helical conveyor and with a slenderness ratio of 4 or greater and a conical part with an angle of from 10 to 25°, and which is operated on the classification principle with the overflow diameter being equal to the sediment discharge diameter ± 10 mm.

4. A process for removing riboflavin from fermentation suspensions as claimed in claim 1, wherein the centrifugation in step c) is carried out in a decanter-type solid-bowl centrifuge with a helical conveyor and with a slenderness ratio of 4 or greater and a conical part with an angle of from 10 to 25°, and which is operated on the classification principle with the differential speed of rotation of the helical conveyor being from ± 0.1 to ± 1 % of the speed of rotation of the bowl and the surface loading being from 0.8 to 1.8 l/(m² · h) for the first decantation and from 0.2 to 0.8 l/(m² h) if the decantation is repeated.

5. A process for removing riboflavin from fermentation suspensions as claimed in claim 4, wherein the surface loading is from 1 to 1.5 l/(m² · h) in the first decantation and from 0.4 to 0.6 l/(m² · h) if the decantation is repeated.

## Revendications

1. Procédé de séparation de riboflavine à partir de suspensions de fermentation par centrifugation, caractérisé en ce que l'on soumet les suspensions de fermentation
a) à un chauffage pendant 1 à 3 heures à une température de 50 à 90°C, puis
b) on refroidit pendant un intervalle de temps de 1 à 10 heures à une température de 0 à 30°C, puis
c) par centrifugation dans une centrifugeuse à décantation, fonctionnant selon le principe d'un trieur, on décompose en une fraction boueuse et une fraction liquide, de manière que la fraction boueuse contienne principalement des cristaux de riboflavine comme solide et la fraction liquide ne contienne pratiquement plus aucune riboflavine à l'état cristallin, et
d) le cas échéant on remet en suspension la fraction boueuse avec 0,5 à 2% en volume d'eau par partie en volume de fraction boueuse et l'on répète une ou plusieurs fois le processus (c).

2. Procédé de séparation de la riboflavine à partir de suspensions de fermentation selon la revendication 1, caractérisé en ce que, à l'étape de réaction c), on décompose les suspensions de fermentation par centrifugation en une fraction boueuse et une fraction liquide, de manière que la partie solide de la fraction boueuse soit composée d'au moins 60 % de cristaux de riboflavine et que la fraction liquide contienne encore une partie importante des composants cellulaires complexes.

3. Procédé de séparation de la riboflavine à partir de suspensions de fermentation selon la revendication 1, caractérisé en ce que, à l'étape de réaction c), on effectue la centrifugation dans une centrifugeuse à vis et à bol plein, du type décanteuse, avec un degré d'élancement égal ou supérieur à 4 et un angle de cône de 10 à 25°C et l'on fonctionne selon le principe d'un trieur, le diamètre de déversement étant égal au diamètre d'évacuation de la boue ± 10 mm.

4. Procédé de séparation de la riboflavine à partir de suspensions de fermentation selon la revendication 1, caractérisé par le fait que, à l'étape de réaction c), on effectue la centrifugation dans une centrifugeuse à vis et bol plein du type décanteuse avec un degré d'élancement égal ou supérieur à 4 et un angle de cône de 10 à 25°C et que l'on opère selon le principe d'un trieur, la vitesse de rotation différentielle de la vis étant de plus ou moins 0,1 à plus ou moins 1% de la vitesse de rotation du tambour et la charge en surface de clarification au premier processus de décantation étant de 0,8 à 1,8 l/(m².h) et, le cas échéant, de 0,2 à 0,8 l/(m².H) en cas de répétition du processus de décantation.

5. Procédé de séparation de la riboflavine à partir de suspensions de fermentation selon la revendication 1, caractérisé en ce que la charge de la surface de clarification lors du premier processus de décantation est de 1 à 1,5 l/(m².h) et, le cas échéant, de 0,4 à 0,6 l/(m².h) en cas de répétition du processus de décantation.
